# EUROPEAN PATENT APPLICATION

(11) **EP 4 567 833 A1**
(43) Date of publication of application: **11.06.2025**
(21) Application number: 23850208.2
(22) Date of filing: 17.05.2023
(51) Int. Cl.: G16H 50/50, G16H 50/70, G16H 10/60, G16H 50/20, G16H 50/30, A61B 5/00, A61B 5/02, A61B 5/026

(54) **METHOD FOR PREDICTING RISK OF BRAIN DISEASE AND METHOD FOR TRAINING RISK ANALYSIS MODEL FOR BRAIN DISEASE**

(30) Priority: 02.08.2022 KR 20220096053
(71) Applicant: Near Brain Inc., Gangnam-gu Seoul 06029 (KR)
(72) Inventor: LEE, Tae Rin, Seoul 03068 (KR)
(74) Representative: Nederlandsch Octrooibureau
(86) International application number: PCT/KR2023/006673
(87) International publication number: WO 2024/029697

(57) **Abstract**

A method for predicting the risk of brain disease according to an embodiment of the present disclosure includes the steps of: acquiring a risk analysis model for brain disease, which has been trained; acquiring target shape information of the brain vessels of the subject patient; acquiring target blood flow information of the brain vessels of the subject patient; acquiring target patient information of the subject patient; and inputting the acquired target shape information, target blood flow information, and target patient information into the risk analysis model for brain disease and acquiring the risk value of brain disease output through the risk analysis model for brain disease.

## Description

### Technical Field

The present disclosure relates to a method of predicting a brain disease risk and a method of training a brain disease risk analysis model. More specifically, the present disclosure relates to a method of predicting a brain disease risk on the basis of cerebrovascular shape information, blood flow information, and/or patient information and a method of training a brain disease risk analysis model for predicting a brain disease risk.

### Background Art

Various imaging techniques, such as positron emission tomography (PET), functional magnetic resonance imaging (fMRI), magnetic resonance angiography (MRA), and computed tomography (CT), have been developed to visualize three-dimensional (3D) structures of a brain and cerebral blood vessels. These advances in imaging technologies have made it possible to measure cerebral blood flow throughout the brain, and with the visualization of cerebral blood vessels and the measurement of cerebral blood flow, research on predicting or diagnosing a brain disease risk on the basis of cerebrovascular shape information and cerebral blood flow information is attracting attention.

Thus far, diagnosing or predicting a brain disease related to brain blood vessels has relied on medical databases of patients' genders, diseases, underlying diseases, races, and the like and the patients' symptoms. However, conventional methods are limited in that they simply predict the possibility of a brain disease on the basis of a patient's symptoms and statistical data of patients, and do not comprehensively consider cerebrovascular shape information and cerebral blood flow information to diagnose or predict a brain disease.

Accordingly, it is necessary to develop a new technology for predicting or analyzing a brain disease risk in comprehensive consideration of cerebrovascular shape information, cerebral blood flow information, and/or a patient's health information.

### Disclosure

### Technical Problem

The present disclosure is directed to providing a method of predicting a brain disease risk on the basis of cerebrovascular shape information, blood flow information, and/or patient information and a method of training a brain disease risk analysis model.

Objects to be achieved by the present disclosure are not limited to that described above, and other objects which have not been described will be clearly understood by those skilled in the technical field to which the present disclosure pertains from the present specification and the accompanying drawings.

### Technical Solution

This summary is provided to introduce a selection of concepts in a simplified form that are further explained in the detailed description below. This summary is not intended to identify the main features or essential features of the claimed subject matter and is not intended to assist in determining the scope of the claimed subject matter.

One aspect of the present disclosure provides a method of predicting a brain disease risk, the method including acquiring a brain disease risk analysis model which has been trained, acquiring target shape information of brain vessels of a subject patient, acquiring target blood flow information of the brain vessels of the subject patient, acquiring target patient information of the subject patient, and inputting the acquired target shape information, target blood flow information, and target patient information into the brain disease risk analysis model and acquiring a brain disease risk value output from the brain disease risk analysis model.

Technical solutions of the present disclosure are not limited to that described above, and other technical solutions which have not been described will be clearly understood by those skilled in the technical field to which the present disclosure pertains from the present specification and the accompanying drawings.

### Advantageous Effects

With a method of training a brain disease risk analysis model according to an embodiment of the present disclosure, it is possible to provide a brain disease risk analysis model for automatically calculating or predicting a brain disease risk on the basis of cerebrovascular shape information data, cerebrovascular blood flow information, and/or a patient's health information.

With a method of predicting a brain disease risk according to an embodiment of the present disclosure, it is possible to calculate a brain disease risk personalized to a subject patient in consideration of the subject patient's cerebrovascular shape information, blood flow information related to the subject patient's blood flow speed and pressure, and patient information related to the subject patient's age, gender, underlying disease, race, and the like.

Effects of the present disclosure are not limited to those described above, and other effects which have not been described will be clearly understood by those skilled in the technical field to which the present disclosure pertains from the present specification and the accompanying drawings.

### Description of Drawings

FIG. 1 is a schematic diagram of a brain disease risk analysis device according to an embodiment of the present disclosure.
FIG. 2 is a schematic diagram illustrating operations of a brain disease risk analysis device according to an embodiment of the present disclosure.
FIG. 3 is a diagram illustrating operational aspects of a brain disease risk analysis device according to an embodiment of the present disclosure.
FIG. 4 is a schematic diagram illustrating operations of a brain disease risk analysis device for training a brain disease risk analysis model according to an embodiment of the present disclosure.
FIG. 5 is a diagram illustrating an aspect of training a brain disease risk analysis model according to an embodiment of the present disclosure.
FIG. 6 is a flowchart illustrating a method of training a brain disease risk analysis model according to an embodiment of the present disclosure.
FIG. 7 is a diagram illustrating an aspect of training a brain disease risk analysis model and an aspect of validating a brain disease risk analysis model according to an embodiment of the present disclosure.
FIG. 8 is a diagram illustrating an aspect of training a brain disease risk analysis model and an aspect of predicting a brain disease risk using a trained brain disease risk analysis model according to an embodiment of the present disclosure.
FIG. 9 is a diagram illustrating an aspect of training a brain disease risk analysis model and an aspect of predicting a brain disease risk using a trained brain disease risk analysis model according to another embodiment of the present disclosure.

### Modes of the Invention

The above-described technical objects, features, and advantages of the present disclosure will be more obvious from the following detailed description in conjunction with the accompanying drawings. However, the present disclosure can be modified in various ways and have several embodiments, and specific embodiments will be illustrated in the drawings and described in detail below.

Throughout the specification, like reference numerals refer to like components in principle. Also, components having the same function within the scope of the same idea illustrated in the drawings of embodiments will be referred to with the same reference numerals, and reiterative description thereof will be omitted.

When detailed description of a known function or element related to the present disclosure is determined to unnecessarily obscure the subject matter of the present disclosure, the detailed description will be omitted. Also, numbers (e.g., first, second, and the like) used in the description process of the present specification are merely identification code for distinguishing one component from others.

In addition, the suffixes "module" and "unit" for components used in the following embodiments are given or used together only to facilitate the writing of the specification. Therefore, these terms do not have distinct meanings or roles therein.

In the following embodiments, singular forms include plural forms unless the context clearly indicates otherwise.

In the following embodiments, the term "include," "have" or the like means that a feature or component described in the specification is present, and it does not preclude the possibility that one or more other features or components are added.

For convenience of description, the sizes of components may be exaggerated or reduced in the accompanying drawings. For example, the size and thickness of each component illustrated in the drawings are arbitrarily indicated for convenience of description, and the present disclosure is not necessarily limited to what is illustrated.

When an embodiment can be implemented differently, a specific process may be performed in a different order from the described order of the process. For example, two processes described in succession may be performed substantially simultaneously, or may be performed in a reverse order to the described order.

In the following embodiments, when components and the like are referred to as being connected, the components may not only be directly connected but may also be indirectly connected with components interposed therebetween.

For example, in the present specification, when components and the like are referred to as being electrically connected, the components may not only be directly electrically connected but may also be indirectly electrically connected with components interposed therebetween.

A method of predicting a brain disease risk according to an embodiment of the present disclosure may include an operation of acquiring a brain disease risk analysis model which has been trained, an operation of acquiring target shape information of brain vessels of a subject patient, an operation of acquiring target blood flow information of the brain vessels of the subject patient, an operation of acquiring target patient information of the subject patient, and an operation of inputting the acquired target shape information, target blood flow information, and target patient information into the brain disease risk analysis model and acquiring a brain disease risk value output from the brain disease risk analysis model.

According to an embodiment of the present disclosure, the operation of acquiring the target shape information may further include an operation of acquiring shape information of a standard blood vessel and an operation of acquiring a difference between the target shape information and the shape information of the standard blood vessel, and the operation of acquiring the brain disease risk value may further include inputting the difference between the target shape information and the shape information of the standard blood vessel into the brain disease risk analysis model and acquiring a brain disease risk value output from the brain disease risk analysis model. The operation of acquiring the target blood flow information may further include an operation of acquiring blood flow information of the standard blood vessel and an operation of acquiring a difference between the target blood flow information and the blood flow information of the standard blood vessel, and the operation of acquiring the brain disease risk value may further include an operation of inputting the difference between the target blood flow information and the blood flow information of the standard blood vessel into the brain disease risk analysis model and acquiring a brain disease risk value output from the brain disease risk analysis model.

According to an embodiment of the present disclosure, the brain disease risk analysis model may be trained on the basis of a first training dataset related to cerebrovascular shape information including cerebrovascular location information or cerebrovascular diameter information, a second training dataset related to cerebrovascular blood flow information including cerebrovascular blood flow speed information or cerebrovascular blood pressure information, a third training dataset related to patient information including patients' ages or genders, and a fourth training dataset related to brain disease information.

According to an embodiment of the present disclosure, the brain disease risk analysis model may be configured to acquire the first training dataset, the second training dataset, the third training dataset, and the fourth training dataset and output a brain disease risk prediction value, and the brain disease risk analysis model may be trained to output the brain disease risk prediction value approximating the brain disease information included in the fourth training dataset.

According to an embodiment of the present disclosure, the brain disease risk analysis model may be trained on the basis of a first training dataset composed of differences between patients' cerebrovascular shape information and standard cerebrovascular shape information, a second training dataset composed of differences between cerebrovascular blood flow information of the patients and standard cerebrovascular blood flow information, a third training dataset related to patient information including the patients' ages or genders, and a fourth training dataset related to brain disease information.

According to an embodiment of the present disclosure, the brain disease risk analysis model may be configured to acquire the first training dataset, the second training dataset, the third training dataset, and the fourth training dataset and output a brain disease risk prediction value, and the brain disease risk analysis model may be trained to output the brain disease risk prediction value approximating the brain disease information included in the fourth training dataset.

According to an embodiment of the present disclosure, a computer-readable recording medium on which a program for executing the method of predicting a brain disease risk is recorded may be provided.

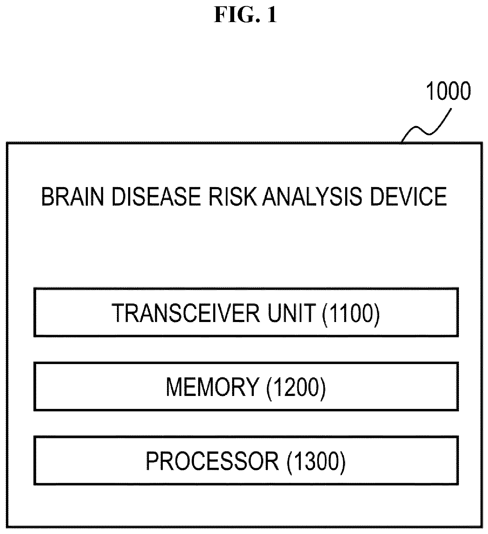

Hereinafter, a method of predicting a brain disease risk, a method of training a brain disease risk analysis model, and a device (or server) for analyzing a brain disease risk (hereinafter, "brain disease risk analysis device") according to embodiments of the present disclosure will be described with reference to FIGS. 1 to 9.

FIG. 1 is a schematic diagram of a brain disease risk analysis device according to an embodiment of the present disclosure.

A brain disease risk analysis device 1000 according to an embodiment of the present disclosure may train a brain disease risk analysis model. Also, the brain disease risk analysis device 1000 may calculate or predict a brain disease risk from a medical image using the brain disease risk analysis model which has been trained.

The brain disease risk analysis device 1000 according to an embodiment of the present disclosure may include a transceiver unit 1100, a memory 1200, and a processor 1300.

The transceiver unit 1100 may communicate with any external device including medical imaging equipment (e.g., magnetic resonance angiography (MRA) equipment, magnetic resonance imaging (MRI) equipment, computed tomography (CT) equipment, positron emission tomography (PET) equipment, and the like). For example, the brain disease risk analysis device 1000 may acquire a captured medical image from the medical imaging equipment through the transceiver unit 1100. Also, the brain disease risk analysis device 1000 may acquire any execution data for executing the brain disease risk analysis model which has been trained, through the transceiver unit 1100. Here, the execution data may be any suitable data for executing the brain disease risk analysis model, including structural information, hierarchy information, and computational libraries of the brain disease risk analysis model and a set of parameters related to weights included in the brain disease risk analysis model. Also, the brain disease risk analysis device 1000 may transmit or output a brain disease risk analysis result acquired through the brain disease risk analysis model to any external device including a user terminal through the transceiver unit 1100.

The brain disease risk analysis device 1000 may access a network through the transceiver unit 1100 to transmit and receive various data. In general, the transceiver unit 1100 may be a wired type transceiver unit or a wireless type transceiver unit. Since the wired-type transceiver unit and the wireless-type transceiver unit each have advantages and disadvantages, the brain disease risk analysis device 1000 may be provided with both the wired-type transceiver unit and the wireless-type transceiver unit in some cases. Here, the wireless-type transceiver unit may mainly use a wireless personal area network (WPAN)-based communication scheme such as Wi-Fi. Alternatively, the wireless-type transceiver unit may use a cellular communication scheme, for example, a Long-Term Evolution (LTE) or fifth generation (5G) communication scheme. However, a wireless communication protocol is not limited to the above examples, and it is possible to use any appropriate wireless-type communication scheme. Representative examples of the wired-type transceiver unit employ local area network (LAN) and Universal Serial Bus (USB) communication, and other communication schemes are also available.

The memory 1200 may store various information. Various data may be temporarily or semi-temporarily stored in the memory 1200. Examples of the memory 1200 may be a hard disk drive (HDD), a solid state drive (SSD), a flash memory, a read-only memory (ROM), a random access memory (RAM), and the like. The memory 1200 may be provided in a form that is embedded into or detachable from the brain disease risk analysis device 1000. Various data necessary for operations of the brain disease risk analysis device 1000, such as an operating system (OS) for operating the brain disease risk analysis device 1000 and a program for operating each element of the brain disease risk analysis device 1000, may be stored in the memory 1200.

The processor 1300 may control overall operations of the brain disease risk analysis device 1000. For example, the processor 1300 may control overall operations of the brain disease risk analysis device 1000, which include an operation of acquiring pixel information of an original medical image, which will be described below, an operation of acquiring coordinate information from the pixel information, an operation of generating cerebrovascular shape information, an operation of acquiring a fluid flow prediction value (or blood flow information), an operation of training the brain disease risk analysis model, and/or an operation of predicting a brain disease risk. Specifically, the processor 1300 may load a program for the overall operations of the brain disease risk analysis device 1000 from the memory 1200 and execute the program. The processor 1300 may be implemented as an application processor (AP), a central processing unit (CPU), or a similar device in accordance with hardware, software, or a combination thereof. As hardware, the processor 1300 may be provided in the form of an electronic circuit for processing an electrical signal to perform a control function, and as software, may be provided in the form of a program or code for operating a hardware circuit.

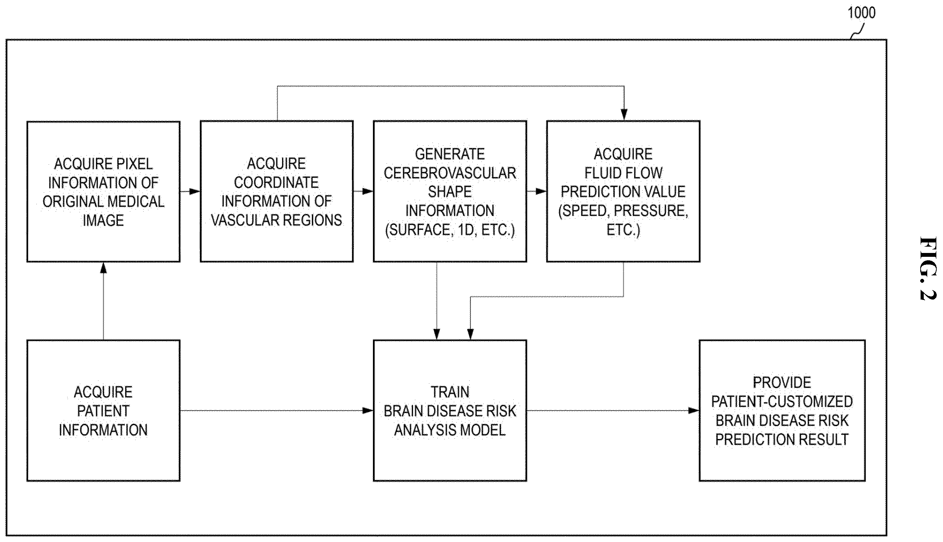

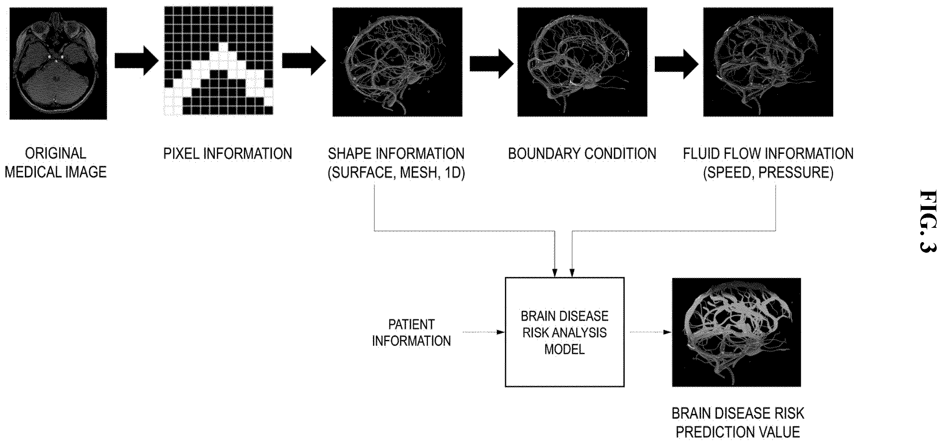

Various operations of the brain disease risk analysis device 1000 according to an embodiment of the present disclosure will be described in detail below with reference to FIGS. 2 and 3. FIG. 2 is a schematic diagram illustrating operations of the brain disease risk analysis device 1000 according to an embodiment of the present disclosure. FIG. 3 is a diagram illustrating operational aspects of the brain disease risk analysis device 1000 according to an embodiment of the present disclosure.

The brain disease risk analysis device 1000 according to an embodiment of the present disclosure may acquire an original medical image. Specifically, the brain disease risk analysis device 1000 may acquire an original medical image (e.g., an MRA image, an MRI image, a CT image, and/or the like) which is acquired through medical imaging equipment. The brain disease risk analysis device 1000 may acquire pixel information from the original medical image. Here, the pixel information may include coordinate information composed of x-coordinates, y-coordinates, and z-coordinates in the original medical image and gray level information of the original medical image. Meanwhile, the brain disease risk analysis device 1000 may acquire patient information stored in an external server (e.g., a hospital server) or a database and acquire pixel information of the original medical image from the original medical image included in the patient information.

The brain disease risk analysis device 1000 according to an embodiment of the present disclosure may acquire coordinate information corresponding to a vascular region from the pixel information of the original medical image. Specifically, the brain disease risk analysis device 1000 may acquire coordinate information related to a region of interest (e.g., a region corresponding to a cerebral blood vessel) from the pixel information of the original medical image in which gray level information has a value equal to or smaller than a predetermined value. As described above, the coordinate information may be the x-coordinates, the y-coordinates, and the z-coordinates of pixels constituting the original medical image. Meanwhile, the coordinate information is related to information on specific points of the original medical image and thus may be referred to as point information or a point cloud.

The brain disease risk analysis device 1000 according to an embodiment of the present disclosure may generate cerebrovascular shape information on the basis of coordinate information corresponding to vascular regions.

For example, the brain disease risk analysis device 1000 may perform an operation of generating a surface model on the basis of the coordinate information corresponding to the vascular regions. Specifically, the brain disease risk analysis device 1000 may generate a surface model that presents cerebrovascular shape information from the coordinate information corresponding to the region of interest using a computer vision segmentation technique. For example, the brain disease risk analysis device 1000 may be implemented to generate a surface model for regions of interest on the basis of coordinates constituting one surface in coordinate information selected from the pixel information. Meanwhile, the original medical image may be composed of a set of two-dimensional (2D) images, thus including three-dimensional (3D) coordinate information. Here, the brain disease risk analysis device 1000 may generate a 3D surface model that presents shape information of a region of interest (e.g., a cerebral blood vessel) on the basis of 3D coordinate information.

As an example, the brain disease risk analysis device 1000 may perform an operation of generating a one-dimensional (1D) model on the basis of the coordinate information corresponding to the vascular region. Specifically, the brain disease risk analysis device 1000 may generate a 1D model that presents shape information of a region of interest (e.g., a cerebral blood vessel) on the basis of coordinate information corresponding to vascular regions. Here, the 1D model is a model that connects points corresponding to the foregoing coordinate information of the region of interest using any method.

As an example, the brain disease risk analysis device 1000 may generate a mesh model on the basis of the coordinate information corresponding to the vascular region. For example, the brain disease risk analysis device 1000 may generate a mesh model from the surface model generated on the basis of the coordinate information.

The brain disease risk analysis device 1000 according an embodiment of the present disclosure may acquire a fluid flow prediction value on the basis of the coordinate information corresponding to the vascular region and cerebrovascular shape information. Specifically, the brain disease risk analysis device 1000 may calculate a fluid flow prediction value (or blood flow information) related to a blood flow speed and/or a blood flow pressure on the basis of the coordinate information corresponding to the vascular region and the cerebrovascular shape information. Further, the brain disease risk analysis device 1000 may acquire a cerebrovascular boundary condition on the basis of the cerebrovascular shape information and calculate or acquire blood flow information related to a blood flow speed and/or a blood flow pressure on the basis of the boundary condition.

As an example, the brain disease risk analysis device 1000 may calculate a fluid flow prediction value from cerebrovascular shape data (e.g., the mesh model and/or the surface model) using computational fluid dynamics (CFD) or computational aided engineering (CAE). As an example, the brain disease risk analysis device 1000 may calculate a fluid flow prediction value from cerebrovascular shape data (e.g., the 1D model) using mathematical expressions in which fluid flow is assumed to be Poiseuille flow.

Meanwhile, the brain disease risk analysis device 1000 according to an embodiment of the present disclosure may train a fluid flow model and perform an operation of predicting or calculating a fluid flow value through the fluid flow model.

As an example, the brain disease risk analysis device 1000 may generate a 1D model from the surface model using a computer vision skeleton technique and generate a fluid flow model from the generated 1D model. Specifically, the brain disease risk analysis device 1000 may train a first neural network that generates a fluid flow model on the basis of a 1D model using an artificial intelligence algorithm, and acquire a fluid flow model from the 1D model through the first neural network which has been trained.

As another example, the brain disease risk analysis device 1000 may generate a fluid flow model on the basis of coordinate information. Specifically, the brain disease risk analysis device 1000 may train a second neural network that generates a fluid flow model on the basis of coordinate information using an artificial intelligence algorithm, and acquire a fluid flow model from the coordinate information through the second neural network which has been trained.

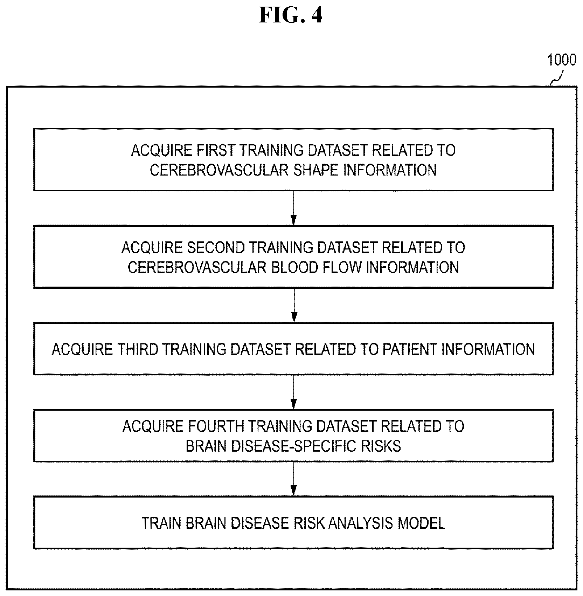

The brain disease risk analysis device 1000 according to an embodiment of the present disclosure may perform an operation of training the brain disease risk analysis model. Specifically, the brain disease risk analysis device 1000 may train the brain disease risk analysis model on the basis of the cerebrovascular shape information, the fluid flow prediction value (or blood flow information), and/or the patient information (e.g., the patient's age, gender, brain disease risk, and the like). Further, the brain disease risk analysis device 1000 according to an embodiment of the present disclosure may acquire a patient-customized brain disease risk prediction result using the brain disease risk analysis model which has been trained and transmitting or outputting the patient-customized brain disease risk prediction result. A method of training the brain disease risk analysis model will be described in detail below with reference to FIGS. 4 to 9.

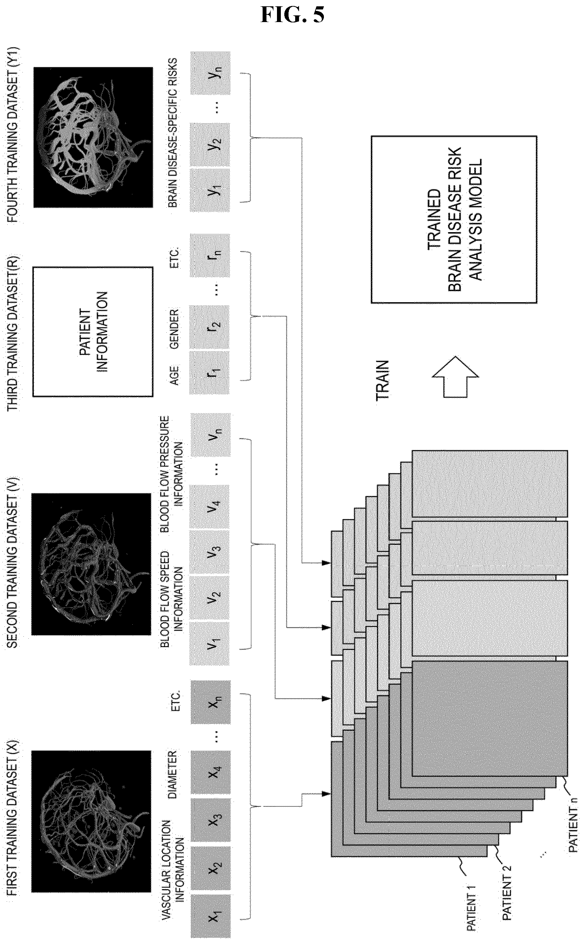

A method of training a brain disease risk analysis model according to an embodiment of the present application will be described in detail below with reference to FIGS. 4 and 5. FIG. 4 is a schematic diagram illustrating operations of the brain disease risk analysis device 1000 for training a brain disease risk analysis model according to an embodiment of the present disclosure. FIG. 5 is a diagram illustrating an aspect of training a brain disease risk analysis model according to an embodiment of the present disclosure.

The brain disease risk analysis device 1000 according to an embodiment of the present disclosure may perform an operation of training a brain disease risk analysis model for calculating a brain disease risk from a medical image. Specifically, the brain disease risk analysis device 1000 may perform an operation of training a brain disease risk analysis model for calculating or quantifying a brain disease risk on the basis of cerebrovascular shape information, cerebrovascular blood flow information, and patient information acquired from the medical image. More specifically, the brain disease risk analysis device 1000 may train a brain disease risk analysis model using a first training dataset X related to vascular shape information xₙ including vascular location information and vascular diameter information, a second training dataset V related to blood flow information vₙ including blood flow speed information and blood flow pressure information, a third training dataset R composed of patient information rₙ related to patients' ages, patients' genders, and/or whether patients have a brain disease, and a fourth training dataset Y1 related to patients' brain disease-specific risks yₙ, and acquire a trained brain disease risk analysis model.

The brain disease risk analysis device 1000 according to an embodiment of the present disclosure may acquire the first training dataset related to cerebrovascular shape information (e.g., vascular location information and/or vascular diameter information) and train the brain disease risk analysis model on the basis of the first training dataset.

As an example, the first training dataset may be composed of each patient's vascular shape information. For example, the first training dataset may include a first patient's first shape information, a second patient's second shape information, and/or an Nth patient's Nth shape information.

As another example, the first training dataset may be composed of differences between patients' vascular shape information and shape information of a standard blood vessel. For example, the first training dataset may be composed of a difference between first shape information of the first patient's blood vessel and the shape information of the standard blood vessel, a difference between second shape information of the second patient's blood vessel and the shape information of the standard blood vessel, and/or a difference between N^{th} shape information of the N^{th} patient's blood vessel and the shape information of the standard blood vessel. Here, the shape information of the standard blood vessel may be shape information of a blood vessel corresponding to a patient's age and gender.

The foregoing description is illustrative, and the brain disease risk analysis model may be trained using the first training dataset that is appropriately generated using any method to achieve the purpose of training the brain disease risk analysis model for predicting a brain disease risk.

The brain disease risk analysis device 1000 according to an embodiment of the present disclosure may acquire the second training dataset related to cerebrovascular blood flow information (e.g., blood flow speed information and blood flow pressure information) and train the brain disease risk analysis model on the basis of the second training dataset.

As an example, the second training dataset may be composed of each patient's vascular blood flow information. For example, the second training dataset may include the first patient's first blood flow information, the second patient's second blood flow information, and/or the Nth patient's Nth blood flow information.

As another example, the second training dataset may be composed of differences between patients' vascular blood flow information and blood flow information of a standard blood vessel. For example, the second training dataset may be composed of a difference between first blood flow information of the first patient's blood vessel and the blood flow information of the standard blood vessel, a difference between second blood flow information of the second patient's blood vessel and the blood flow information of the standard blood vessel, and/or a difference between N^{th} blood flow information of the N^{th} patient's blood vessel and the blood flow information of the standard blood vessel. Here, the standard blood vessel may be a standard blood vessel corresponding to a patient's gender and age.

The foregoing description is illustrative, and the brain disease risk analysis model may be trained using the second training dataset that is appropriately generated using any method to achieve the purpose of training the brain disease risk analysis model for predicting a brain disease risk.

The brain disease risk analysis device 1000 according to an embodiment of the present disclosure may acquire the third training dataset related to patient information and train the brain disease risk analysis model on the basis of the third training dataset. Specifically, the third training dataset may be composed of patient data about at least one of patients' ages, genders, underlying diseases, and races. Specifically, the brain disease risk analysis device 1000 may acquire the third training dataset on the basis of patient information acquired from an external server (e.g., a hospital server).

The brain disease risk analysis device 1000 according to an embodiment of the present disclosure may acquire the fourth training dataset related to brain disease-specific risk information and train the brain disease risk analysis model using the fourth training dataset. For example, the brain disease risk analysis device 1000 may assign brain disease risk information (e.g., a brain disease possibility value) on the basis of location information included in vascular shape information and brain disease information included in patient information. More specifically, the brain disease risk analysis device 1000 may detect a brain region related to a brain disease on the basis of the brain disease information included in the patient information and assign brain disease risk information (e.g., a brain disease possibility value) to location information corresponding to the detected brain region, thereby acquiring the fourth training dataset. As an example, the brain disease risk analysis device 1000 may calculate a possibility value of a brain disease occurring in each brain region on the basis of the patient information and assign the calculated possibility value (e.g., a value between 0 and 1) to the brain region using location information included in vascular shape information. For example, in the case of a patient suffering from a stroke, the brain disease risk analysis device 1000 may detect a brain region in which the stroke has actually occurred on the basis of patient information and assign stroke risk information (e.g., a risk of 1) indicating that the stroke has occurred to location information corresponding to the detected brain region. For example, the brain disease risk analysis device 1000 may detect a brain region in which no stroke has occurred on the basis of the patient information and assign stroke risk information (e.g., a risk of 0) indicating that no stroke has occurred to location information corresponding to the detected brain region.

The brain disease risk analysis device 1000 according to an embodiment of the present disclosure may train the brain disease risk analysis model on the basis of a first training dataset related to vascular shape information, a second training dataset related to blood flow information, a third training dataset related to patient information, and a fourth training dataset related to brain disease-specific risk information.

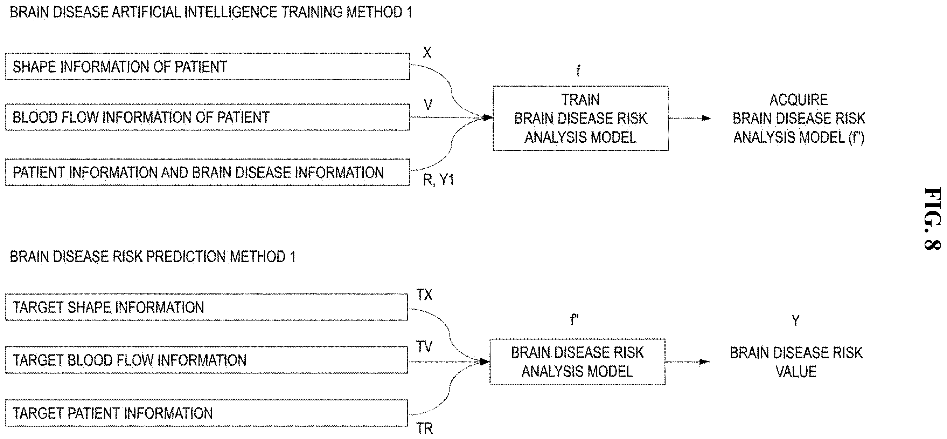

As an example, the brain disease risk analysis device 1000 may train the brain disease risk analysis model on the basis of patients' cerebrovascular data (e.g., cerebrovascular shape information or cerebrovascular blood flow information). This will be described in further detail below with reference to FIG. 8.

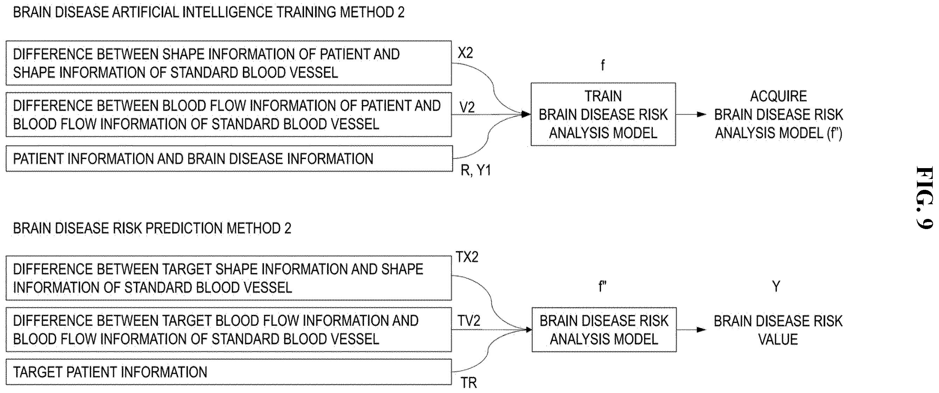

As another example, the brain disease risk analysis device 1000 may train the brain disease risk analysis model on the basis of patients' cerebrovascular data (e.g., cerebrovascular shape information or cerebrovascular blood flow information) and cerebrovascular data of the standard blood vessel (e.g., shape information of the standard blood vessel or blood flow information of the standard blood vessel). Specifically, the brain disease risk analysis device 1000 may train the brain disease risk analysis model on the basis of differences between patients' cerebrovascular data (e.g., cerebrovascular shape information or cerebrovascular blood flow information) and cerebrovascular data of the standard blood vessel (e.g., the shape information of the standard blood vessel or blood flow information of the standard blood vessel). For example, the brain disease risk analysis device 1000 may train the brain disease risk analysis model using a training dataset related to differences between patients' cerebrovascular shape information and the shape information of the standard blood vessel. For example, the brain disease risk analysis device 1000 may train the brain disease risk analysis model using a training dataset related to differences between patients' cerebrovascular blood flow information and the blood flow information of the standard blood vessel. This will be described in further detail with reference to FIG. 9.

It has been described in FIGS. 4 and 5 that a brain disease risk analysis model is a single model. However, this is only for convenience of description and should not be construed as limiting. Individual models may be trained on the basis of different training datasets, and a brain disease risk may be calculated in an integrated manner through a structure in which some layers of models are shared.

For example, a first brain disease risk analysis model may be configured to acquire the first training dataset and the fourth training dataset related to brain disease-specific risks acquired from patient information and output a brain disease risk prediction value. Here, the first brain disease risk analysis model may be trained when any parameters included in the first brain disease risk analysis model are updated on the basis of the brain disease risk prediction value and the fourth training dataset related to the brain disease-specific risks.

For example, a second brain disease risk analysis model may be configured to acquire the second training dataset and the fourth training dataset related to the brain disease-specific risks acquired from the patient information and output a brain disease risk prediction value. Here, the second brain disease risk analysis model may be trained when any parameters included in the second brain disease risk analysis model are updated on the basis of the brain disease risk prediction value and the fourth training dataset related to the brain disease-specific risks.

Some layers of the first brain disease risk analysis model and some layers of the second brain disease risk analysis model may be shared with each other, and it is possible to predict a brain disease risk in consideration of both cerebrovascular shape information and cerebrovascular blood flow information using the structure.

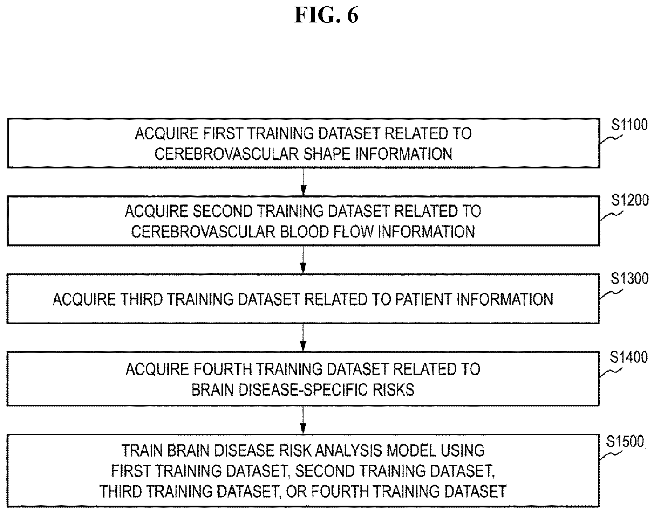

A method of training a brain disease risk analysis model and a method of predicting a brain disease risk according to an embodiment of the present disclosure will be described below with reference to FIGS. 6 to 9. FIG. 6 is a flowchart illustrating a method of training a brain disease risk analysis model according to an embodiment of the present disclosure. In describing the method of training a brain disease risk analysis model, some embodiments overlapping the description of FIGS. 2 to 5 may be omitted. However, this is only for convenience of description and should not be construed as limiting.

The method of training a brain disease risk analysis model according to an embodiment of the present disclosure may include an operation S1100 of acquiring a first training dataset related to cerebrovascular shape information, an operation S1200 of acquiring a second training dataset related to cerebrovascular blood flow information, an operation S1300 of acquiring a third training dataset related to patient information, an operation S1400 of acquiring a fourth training dataset related to brain disease-specific risks, and an operation S1500 of training a brain disease risk analysis model using the first training dataset, the second training dataset, the third training dataset, or the fourth training dataset.

In the operation S1100 of acquiring the first training dataset related to cerebrovascular shape information, the brain disease risk analysis device 1000 may acquire a first training dataset X related to cerebrovascular shape information (e.g., vascular location information and vascular diameter information). As an example, the first training dataset may be composed of each user's vascular shape information as described above. For example, the first training dataset X may include a first patient's first shape information, a second patient's second shape information, and/or an N^{th} patient's N^{th} shape information. As another example, the first training dataset X may be composed of a difference between a patient's vascular shape information and shape information of a standard blood vessel. For example, the first training dataset may be composed of a difference between first shape information of the first patient's blood vessel and the shape information of the standard blood vessel, a difference between second shape information of the second patient's blood vessel and the shape information of the standard blood vessel, and/or a difference between N^{th} shape information of the N^{th} patient's blood vessel and the shape information of the standard blood vessel.

In the operation S1200 of acquiring the second training dataset related to cerebrovascular blood flow information, the brain disease risk analysis device 1000 may acquire a second training dataset related to cerebrovascular blood flow information (e.g., blood flow speed information and blood flow pressure information). As an example, the second training dataset may be composed of each user's vascular blood flow information. For example, the second training dataset may include the first patient's first blood flow information, the second patient's second blood flow information, and/or the Nth patient's Nth blood flow information. As another example, the second training dataset may be composed of differences between vascular blood flow information of patients and the blood flow information of the standard blood vessel. For example, the second training dataset may include a difference between first blood flow information of the first patient's blood vessel and the blood flow information of the standard blood vessel, a difference between second blood flow information of the second patient's blood vessel and the blood flow information of the standard blood vessel, and/or a difference between Nth blood flow information of the Nth patient's blood vessel and the blood flow information of the standard blood vessel.

In the operation S1300 of acquiring the third training dataset related to patient information, the brain disease risk analysis device 1000 may acquire a third training dataset related to patient information (e.g., patients' ages, genders, underlying diseases, and/or races).

In the operation S1400 of acquiring the fourth training dataset related to brain disease-specific risks, the brain disease risk analysis device 1000 may acquire a fourth training dataset related to brain disease-specific risk information. As described above, the fourth training dataset may be related to brain disease-specific risk information (e.g., a brain disease possibility value) that is assigned on the basis of the location information included in the vascular shape information and brain disease information included in the patient information. Specifically, the brain disease risk analysis device 1000 may calculate a possibility value of a brain disease occurring in each brain region on the basis of the patient information and assign the calculated possibility value (e.g., a value between 0 and 1) to the brain region using location information included in the vascular shape information, thereby acquiring the fourth training dataset.

In the operation S1500 of training the brain disease risk analysis model using the first training dataset, the second training dataset, the third training dataset, or the fourth training dataset, the brain disease risk analysis device 1000 may train the brain disease risk analysis model on the basis of the first training dataset related to vascular shape information, the second training dataset related to vascular blood flow information, the third training dataset related to patient information, and the fourth training dataset related to brain disease-specific risks. Specifically, the brain disease risk analysis device 1000 may train the brain disease risk analysis model on the basis of a prediction value which is output from the brain disease risk analysis model, and brain disease-specific risks included in the fourth training dataset. For example, the brain disease risk analysis device 1000 may train the brain disease risk analysis model by updating parameters included in the brain disease risk analysis model such that a prediction value output from the brain disease risk analysis model approximates a brain disease-specific risk included in the fourth training dataset.

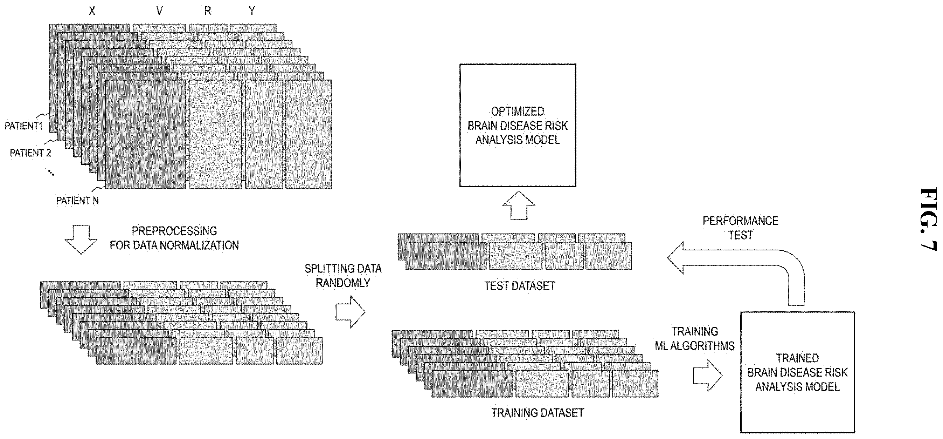

FIG. 7 will be described below. FIG. 7 is a diagram illustrating an aspect of training a brain disease risk analysis model and an aspect of validating a brain disease risk analysis model according to an embodiment of the present disclosure. The brain disease risk analysis device 1000 according to an embodiment of the present disclosure may perform an operation of preprocessing training data composed of a first training dataset, a second training dataset, a third training dataset, and/or a fourth training dataset. For example, the brain disease risk analysis device 1000 may normalize each piece of data included in the training data composed of the first training dataset, the second training dataset, the third training dataset, and/or the fourth training dataset.

Further, the brain disease risk analysis device 1000 may perform an operation of splitting the preprocessed training data into a training dataset and a test dataset or validation dataset. For example, the brain disease risk analysis device 1000 may randomly split the preprocessed training data into a training dataset and a test dataset. Here, the brain disease risk analysis device 1000 may train a brain disease risk analysis model through any machine learning (ML) algorithm using the training dataset. The brain disease risk analysis device 1000 may test or validate the performance of the trained brain disease risk analysis model using the validation dataset. For example, the brain disease risk analysis device 1000 may calculate the performance of the trained brain disease risk analysis model, compare the calculated performance with predetermined target performance, and train the brain disease risk analysis model as described above until the calculated performance becomes higher than the predetermined target performance, thereby acquiring an optimized brain disease risk analysis model.

FIG. 8 is a diagram illustrating an aspect of training a brain disease risk analysis model and an aspect of predicting a brain disease risk using a trained brain disease risk analysis model according to an embodiment of the present disclosure.

The brain disease risk analysis device 1000 according to an embodiment of the present disclosure may train a brain disease risk analysis model f on the basis of a first training dataset X related to patients' vascular shape information acquired from the patients' vascular images, a second training dataset V related to blood flow information of the patients, a third training dataset R related to patient information, and a fourth training dataset Y1 related to brain disease-specific risk information acquired from the patient information. Specifically, the brain disease risk analysis device 1000 may input the first training dataset X, the second training dataset V, the third training dataset R, and the fourth training dataset Y1 to the brain disease risk analysis model f and acquire a brain disease risk prediction value output from the brain disease risk analysis model f. Here, the brain disease risk analysis device 1000 may update any parameters included in the brain disease risk analysis model on the basis of the brain disease risk prediction value and the brain disease-specific risk information. Specifically, the brain disease risk analysis device 1000 may compare the brain disease risk prediction value with the brain disease-specific risk information Y1 and train the brain disease risk analysis model f by updating any parameters included in the brain disease risk analysis model f such that a brain disease risk prediction value approximating the brain disease-specific risk information may be output, thereby acquiring a brain disease risk analysis model f" which has been trained.

Further, the brain disease risk analysis device 1000 according to an embodiment of the present disclosure may predict a brain disease risk using the trained brain disease risk analysis model f". Specifically, the brain disease risk analysis device 1000 may be configured to acquire target shape information TX from a vascular image of a subject patient to be analyzed, acquire target blood flow information TV of the subject patient and target patient information TR of the subject patient, and input the target shape information TX, the target blood flow information TV, and the target patient information TR to the trained brain disease risk analysis model f". Here, the brain disease risk analysis device 1000 may acquire a brain disease risk value Y output from the trained brain disease risk analysis model f'. As described above, since the trained brain disease risk analysis model f" has been trained to output the brain disease-specific risk information Y1 on the basis of the vascular shape information X, the vascular blood flow information V, and the patient information R, it is possible to output the brain disease risk value Y on the basis of the target shape information TX, the target blood flow information TV, and the target patient information TR of the subject patient.

FIG. 9 is a diagram illustrating an aspect of training a brain disease risk analysis model and an aspect of predicting a brain disease risk using a trained brain disease risk analysis model according to another embodiment of the present disclosure.

The brain disease risk analysis device 1000 according to the other embodiment of the present disclosure may train a brain disease risk analysis model f on the basis of a first training dataset X2 composed of differences between patients' vascular shape information acquired from the patients' vascular images and shape information of a standard blood vessel, a second training dataset V2 composed of differences between blood flow information of the patients and blood flow information of the standard blood vessel, a third training dataset R related to patient information, and a fourth training dataset Y1 related to brain disease-specific risk information acquired from the patient information. Specifically, the brain disease risk analysis device 1000 may input the first training dataset X2, the second training dataset V2, the third training dataset R, and the fourth training dataset Y1 to the brain disease risk analysis model f and acquire a brain disease risk prediction value output from the brain disease risk analysis model f. Here, the brain disease risk analysis device 1000 may update any parameters included in the brain disease risk analysis model on the basis of the brain disease risk prediction value and the brain disease-specific risk information Y1. Specifically, the brain disease risk analysis device 1000 may compare the brain disease risk prediction value with the brain disease-specific risk information Y1 and train the brain disease risk analysis model f by updating any parameters included in the brain disease risk analysis model f such that a brain disease risk prediction value approximating the brain disease-specific risk information Y1 may be output, thereby acquiring a brain disease risk analysis model f" which has been trained.

Further, the brain disease risk analysis device 1000 according to an embodiment of the present disclosure may predict a brain disease risk using the trained brain disease risk analysis model f". Specifically, the brain disease risk analysis device 1000 may be configured to acquire a difference TX2 between target shape information acquired from a vascular image of a subject patient to be analyzed and shape information of a standard blood vessel, a difference TV2 between target blood flow information of the subject patient and blood flow information of the standard blood vessel, and target patient information TR of the subject patient, and input the difference TX2 between the target shape information and the shape information of the standard blood vessel, the difference TV2 between the target blood flow information and the blood flow information of the standard blood vessel, and the target patient information TR to the trained brain disease risk analysis model f". Here, the brain disease risk analysis device 1000 may acquire a brain disease risk value Y output from the trained brain disease risk analysis model f". As described above, since the trained brain disease risk analysis model f" has been trained to output the brain disease-specific risk information on the basis of a first training dataset composed of the difference X2 between vascular shape information and the shape information of the standard blood vessel, a second training dataset composed of the difference V2 between vascular blood flow information and the blood flow information of the standard blood vessel, and the patient information R, it is possible to output the brain disease risk value Y on the basis of the difference TX2 between the target shape information and the shape information of the standard blood vessel, the difference TV2 between the target blood flow information and the blood flow information of the standard blood vessel, and the target patient information TR of the subject patient.

The trained brain disease risk analysis model f" can provide the effect of outputting a patient-customized brain disease risk personalized to a subject patient in consideration of the subject patient's cerebrovascular shape information, blood flow information related to the subject patient's blood flow speed and pressure, and patient information related to the subject patient's age, gender, underlying disease, race, and the like.

Meanwhile, predicting a brain disease risk on the basis of a patient's cerebrovascular data (e.g., cerebrovascular shape information or cerebrovascular blood flow information) has been mainly described in FIG. 8, and predicting a brain disease risk on the basis of a difference between a patient's cerebrovascular data and standard cerebrovascular data has been mainly described in FIG. 9. However, these are illustrative and may be selected, combined, or changed using any appropriate method.

With a method of training a brain disease risk analysis model according to an embodiment of the present disclosure, it is possible to provide a brain disease risk analysis model for automatically calculating or predicting a brain disease risk on the basis of cerebrovascular shape information, cerebrovascular blood flow information, and/or a patient information.

The various operations of the brain disease risk analysis device 1000 described above may be stored in the memory 1200 of the brain disease risk analysis device 1000, and the processor 1300 of the brain disease risk analysis device 1000 may perform the operations stored in the memory 1200.

The features, structures, effects, and the like described in the above embodiments are included in at least one embodiment of the present disclosure and are not necessarily limited to only one embodiment. Further, the features, structures, effects, and the like illustrated in each embodiment can be combined or modified for implementation in other embodiments by those of ordinary skill in the art to which the embodiments pertain. Accordingly, the contents related to such combinations and modifications should be construed as falling within the scope of the present disclosure.

Although embodiments have been mainly described above, these are merely illustrative and do not limit the present disclosure. Those skilled in the art to which the present disclosure pertains should understand that several modifications and applications that have not been described above can be made without departing from the fundamental characteristics of the present embodiments. In other words, each component specifically presented in the embodiments may be modified for implementation. In addition, differences associated with these modifications and applications are to be construed as falling within the scope of the present disclosure defined by the appended claims.

### Industrial Applicability

The above-described method of predicting a brain disease risk, method of training a brain disease risk analysis model, and brain disease risk analysis device can be applied to the field of medical service for providing a medical service.

## Claims

1. A method of predicting a brain disease risk by a brain disease risk analysis device, the method comprising:
acquiring a brain disease risk analysis model which has been trained;
acquiring target shape information of brain vessels of a subject patient;
acquiring target blood flow information of the brain vessels of the subject patient;
acquiring target patient information of the subject patient; and
inputting the acquired target shape information, target blood flow information, and target patient information into the brain disease risk analysis model and acquiring a brain disease risk value output from the brain disease risk analysis model.

2. The method of claim 1, wherein the acquiring of the target shape information further comprises:
acquiring shape information of a standard blood vessel; and
acquiring a difference between the target shape information and the shape information of the standard blood vessel, and
the acquiring of the brain disease risk value further comprises inputting the difference between the target shape information and the shape information of the standard blood vessel into the brain disease risk analysis model and acquiring a brain disease risk value output from the brain disease risk analysis model.

3. The method of claim 2, wherein the acquiring of the target blood flow information further comprises:
acquiring blood flow information of the standard blood vessel; and
acquiring a difference between the target blood flow information and the blood flow information of the standard blood vessel, and
the acquiring of the brain disease risk value further comprises inputting the difference between the target blood flow information and the blood flow information of the standard blood vessel into the brain disease risk analysis model and acquiring a brain disease risk value output from the brain disease risk analysis model.

4. The method of claim 1, wherein the brain disease risk analysis model is trained on the basis of a first training dataset related to cerebrovascular shape information including cerebrovascular location information or cerebrovascular diameter information, a second training dataset related to cerebrovascular blood flow information including cerebrovascular blood flow speed information or cerebrovascular blood pressure information, a third training dataset related to patient information including patients' ages or genders, and a fourth training dataset related to brain disease information.

5. The method of claim 4, wherein the brain disease risk analysis model is configured to acquire the first training dataset, the second training dataset, the third training dataset, and the fourth training dataset and output a brain disease risk prediction value, and
the brain disease risk analysis model is trained to output the brain disease risk prediction value approximating the brain disease information included in the fourth training dataset.

6. The method of claim 3, wherein the brain disease risk analysis model is trained on the basis of a first training dataset composed of differences between patients' cerebrovascular shape information and standard cerebrovascular shape information, a second training dataset composed of differences between cerebrovascular blood flow information of the patients and standard cerebrovascular blood flow information, a third training dataset related to the patient information including patients' ages or genders, and a fourth training dataset related to brain disease information.

7. The method of claim 6, wherein the brain disease risk analysis model is configured to acquire the first training dataset, the second training dataset, the third training dataset, and the fourth training dataset and output a brain disease risk prediction value, and
the brain disease risk analysis model is trained to output the brain disease risk prediction value approximating the brain disease information included in the fourth training dataset.

8. A computer-readable recording medium on which a program for causing a computer to execute the method of any one of claims 1 to 7 is recorded.
